# EUROPÄISCHE PATENTSCHRIFT

(11) **EP 2 258 323 B1**
(45) Veröffentlichungstag und Bekanntmachung des Hinweises auf die Patenterteilung: **09.01.2019**
(21) Anmeldenummer: 10157040.6
(22) Anmeldetag: 19.03.2010
(51) Int. Cl.: A61F 2/958

(54) **Applikationsvorrichtung für Stents mit funktional strukturierter Oberfläche**
Implantation device for stents with a functionally structured surface
Dispositif d'implantation pour stents à surface structurée fonctionnelle

(30) Priorität: 04.06.2009 US 183991 P; 05.06.2009 US 184321 P
(43) Veröffentlichungstag der Anmeldung: 08.12.2010
(73) Patentinhaber: BIOTRONIK AG, 8180 Bülach (CH)
(72) Erfinder: Adden, Nina, 90427, Nürnberg (DE); Surber, Bettina, 8542 Gachnang (CH)
(74) Vertreter: Keck, Hans-Georg

(56) Entgegenhaltungen:
- US-A1- 2006 212 106
- US-A1- 2009 112 239

## Beschreibung

### Technisches Gebiet

Die vorliegende Erfindung betrifft das Gebiet der Applikationsvorrichtungen für Stents.

### Technologischer Hintergrund und Stand der Technik

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu einen rohrförmigen Grundkörper mit einer filigranen Tragstruktur aus metallischen Streben auf, der zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen eine Umfangswandung von ausreichender Tragkraft, um das verengte Gefäß im gewünschten Maße offen zu halten, und einen rohrförmigen Grundkörper, durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe einer Applikationsvorrichtung, beispielsweise eines Katheters, derart zu applizieren, dass das Gefäß den gewünschten, vergrößerten Innendurchmesser aufweist.

Zur Applikation von Stents können unterschiedliche Applikationsvorrichtungen genutzt werden.

Zur Applikation nicht selbstexpandierbarer Stents werden meist Ballonkatheter verwendet. Dabei wird der Stent auf eine Oberfläche des nicht-dilatierten Ballons meist durch sogenanntes "crimpen" fixiert. Nachdem der mit dem Stent beladene Ballonkatheter in das Gefäß eingeführt wurde und der Stent an der Stelle angelangt ist, an der er appliziert werden soll, wird der Ballon dilatiert. Dadurch wird der Stent geweitet und mit der Gefäßwand in Kontakt gebracht. Nachdem der Stent ausreichend geweitet und das Gefäß ausreichend aufgedehnt wurde, wird der Druck im Ballon verringert, der Ballon kehrt in einen nicht-dilatierten Zustand zurück und kann wieder entfernt werden, während der applizierte Stent im Gefäß verbleibt und dieses offen hält. Geeignete Ballonkatheter zur Stentapplikation sind bekannt und beispielhaft in DE 102 15 462 beschrieben.

Alternativ kann ein selbstexpandierendes Stentdesign verwendet werden. Applikationsvorrichtungen zur Applikation selbstexpandierender Stents weisen in der Regel keinen dilatierbaren Ballon zur Stentapplikation auf. Hier werden meist Kathetervorrichtungen verwendet, die einen Innenschlauch und einen Außenschlauch aufweisen, wobei der zu applizierende Stent zunächst auf einer Oberfläche des Innenschlauchs gecrimpt vorliegt und von einem darüber geschobenen Außenschlauch bedeckt ist. Selbstexpandierende Stents weisen in gecrimpter Form eine radiale Kraft auf, die die Stentaußenfläche an die den Stent abdeckende Innenfläche des Außenschlauchs drückt. Der Katheter wird in das Gefäß eingeführt, bis der Stent an der Stelle zu liegen kommt, an der er appliziert werden soll. Zur Applikation wird nun der Außenschlauch zurückgezogen, bis der gesamte Stent freigelegt ist. Durch die radiale Kraft expandiert der Stent eigenständig, löst sich vom Innenschlauch und tritt mit der Gefäßwand in Kontakt. Nachdem sich der Stent ausreichend geweitet hat, kann der Katheter wieder entfernt werden, während der applizierte Stent im Gefäß verbleibt und dieses offen hält. Geeignete Katheter zur Applikation von selbstexpandierbaren Stents sind bekannt und beispielhaft in US 5,824,041 beschrieben.

Sowohl bei der Applikation mittels Ballonkatheter als auch bei der Applikation von selbstexpandierbaren Stents ist es wesentlich, dass zwischen gecrimptem Stent und der Oberfläche der Applikationsvorrichtung, auf welcher der Stent gecrimpt vorliegt, eine ausreichende Haltekraft zustande kommt.

Insbesondere muss ein selbstexpandierbarer Stent auf dem Innenschlauch derart fixiert vorliegen, dass er beim Zurückziehen des Außenschlauchs während des Applikationsvorgangs nicht verrutscht.

Im Stand der Technik wird dies meist dadurch erreicht, dass ein sogenannter Stentstopper verwendet wird, der proximal vom Stent auf dem Innenschlauch angebracht wird. Bei dieser Lösung wird aber beim Freisetzen der Stent durch eine erhöhte Reibung in sich zusammengeschoben, was die Stenteigenschaften beeinträchtigen kann.

Bei der Applikation mittels Ballonkatheter, muss der Stent auf der nicht-dilatierten Ballonoberfläche fixiert vorliegen. Dies ist nicht immer für alle Stentarten in ausreichendem Maße möglich. So können z.B. wirkstofffreisetzende Stents, Stents mit einer großen Strutwandstärke oder Stents mit großer Strutbreite häufig nicht ausreichend durch einfaches Crimpen fixiert werden.

In solchen Fällen ist es bislang notwendig, eine zusätzliche Stentfixierung vorzunehmen, die nachträglich über dem gecrimpten Stent aufgebracht werden muss, was meist maschinell nicht möglich ist. Zudem können diese Stentfixierungen das Aufweitverhalten des Stents beeinflussen und/oder im Fall von beschichteten Stents die Beschichtung beeinträchtigen. Ferner wird in US 2009/0112239 A1 ein Ballonkatheter beschrieben, der eine Ballonoberfläche mit Strukturen aufweist, die zur Verhinderung von Embolien beiträgt.

Aufgabe der vorliegenden Erfindung ist es, einen oder mehrere Nachteile des Standes der Technik zu mindern oder zu verhindern. Insbesondere sollen Applikationsvorrichtungen für Stents bereitgestellt werden, die weniger zusätzliche Maßnahmen zur Stentfixierung erfordern.

### Erfindungsgemäße Lösung

Die Aufgabe wird gelöst durch Bereitstellung einer Applikationsvorrichtung für Stents, die, mindestens auf einem Teil der nach außen gewandten Oberfläche des Bereichs der Vorrichtung haarähnliche Fortsätze aufweist, der zum Anbringen oder Crimpen des zu applizierenden Stents vorgesehen ist, wobei der durchschnittliche Durchmesser *D*, die durchschnittliche Länge *L* und der gemittelte Zentrum-zu-Zentrum-Abstand *P* der haarähnlichen Fortsätze zueinander derart ausgewählt sind, dass ein Stent auf der mit haarähnlichen Fortsätzen bedeckten Oberfläche im Wesentlichen mittels van der Waals-Kräften fixierbar ist.

Angelehnt an die Oberflächenstruktur der Innenfläche von Geckofüßen, weist eine Oberfläche der Applikationsvorrichtung eine Vielzahl von haarähnlichen Fortsätzen auf. Der Gecko kann durch eine Vielzahl an sub-mikrometer großen Härchen an seinen Füßen auch glatte Flächen senkrecht oder auch kopfüber hoch laufen. Dieser Effekt ist eine Kombination von Kapillarkräften und van der Waals-Kräften. Bei der erfindungsgemäßen Applikationsvorrichtung werden insbesondere die van der Waals-Kräfte einer solchen Oberflächenstruktur genutzt, um einen gecrimpten Stent auf einer Oberfläche der Applikationsvorrichtung zu fixieren und erst bei Bedarf wieder freizusetzen. Diese Oberflächenstrukturierung erlaubt es, auf Maßnahmen zur Stentfixierung, die sich an ein Crimpen anschließen, zu verzichten. Durch Kontakt der genannten Oberflächenstruktur der Applikationsvorrichtung mit einer Innenfläche des Stents wird der Stent im Wesentlichen durch van der Waals-Kräfte auf der Applikationsvorrichtung fixiert. Die Summe der van der Waals-Kräfte pro haarähnlichem Fortsatz ergibt zusammen eine ausreichende Haltekraft für den Stent. Dabei sind die van der Waals-Kräfte zwischen der Innenfläche des Stents und den haarähnlichen Fortsätzen dann am größten, wenn die Kraft auf die Kontaktfläche der haarähnlichen Fortsätze senkrecht wirkt, wie im Fall des Einführens des mit einem Stent versehenen Ballonkatheters in ein Gefäß. Es ist kein aufwändiges weiteres Stentfixierungsverfahren mehr notwendig, um einen Stent nach dem Crimpen auf der Applikationsvorrichtung zu befestigen.

Grundsätzlich kann jede Applikationsvorrichtung für Stents eingesetzt werden, vorausgesetzt eine Oberfläche des zu applizierenden Stents steht vor der Applikation mit einer Oberfläche der Applikationsvorrichtung in Kontakt. Bevorzugt umfasst die Applikationsvorrichtung einen Katheter.

Die Applikationsvorrichtung kann eine dilatierbare Ballonoberfläche umfassen, auf die ein Stent gecrimpt werden kann, wie sie beispielsweise ein Ballonkatheter aufweist. Grundsätzlich kann für den erfindungsgemäßen Ballonkatheter jedes bekannte Ballonkathetersystem verwendet werden. Insbesondere kann ein Ballonkatheter verwendet werden mit einem Innenschaft, an dem ein distales Ende eines expandierbaren Ballons befestigt ist, der in einem nicht-expandierten, deflatierten Zustand an einer Außenoberfläche des Innenschaftes zumindest teilweise anliegt. Die Erfindung betrifft insbesondere solche Katheter, die auf der Außenseite des deflatierten Ballons einen Stent tragen können, der nach Einführen in ein Gefäß durch Inflation des Ballons mit einem Fluid expandiert und so am vorgesehenen Ort an eine Gefäßwand gedrückt wird.

Ballonkatheter der vorgesehenen Art weisen üblicherweise neben einem Innenschaft und dem Ballon auch einen Außenschaft auf, der wenigstens bis zu einem proximalen Ende des Ballons reicht und mit diesem fluiddicht verbunden ist. Zwischen Innen- und Außenschaft des Katheters ist üblicherweise eine in Längsrichtung des Katheters von seinem proximalen Ende bis ins Innere des Ballons reichende Fluidleitung vorgesehen, die sich beispielsweise daraus ergibt, dass der Außenschaft einen Innendurchmesser besitzt, der größer ist, als ein Außendurchmesser des Innenschaftes.

Im Inneren des Innenschaftes ist ein vom Innenschaft eingeschlossener, sich in Längsrichtung des Innenschaftes erstreckender Hohlraum als Lumen vorgesehen. Dieses Lumen dient beispielsweise der Aufnahme eines Mandrins oder eines Führungsdrahtes. Katheter und Führungsdraht sind dann beispielsweise so ausgebildet, dass der Führungsdraht an der distalen Spitze des Katheters austreten kann und vom proximalen Ende her zu steuern ist. Der Führungsdraht wird zum Beispiel mit Hilfe von Steuermitteln so ausgelenkt, dass er auch in abzweigende Blutgefässe leicht einzuführen ist. Der Ballonkatheter kann dann entlang des Führungsdrahtes nachgeschoben werden.

Unabhängig von der Art des Katheters, insbesondere hinsichtlich der Gestaltung der Führungsmittel, weisen Ballonkatheter an ihrem distalen Ende den bereits erwähnten, expandierbaren Ballon auf. Während des Einführens des Ballonkatheters ist der Ballon komprimiert und liegt eng am Innenschaft des Katheters an. Durch Inflatieren des Ballons mit einem Fluid kann dieser expandiert werden. Dieses Expandieren des Ballons geschieht, sobald der Ballon bis zu der bestimmungsgemäßen Position geführt ist. Durch das Expandieren des Ballons wird der Stent plastisch verformt, so dass er sich dauerhaft an eine Gefäßwand anlagern kann.

Die erfindungsgemäße Applikationsvorrichtung kann alternativ als Vorrichtung zur Applikation von selbstexpandierbaren Stents ausgestaltet sein, beispielsweise eine Oberfläche zur Applikation selbstexpandierbarer Stents aufweisen. Vorrichtungen zur Applikation selbstexpandierbarer Stents sind dadurch gekennzeichnet, dass keine Mittel zur aktiven plastischen Verformung eines aufgecrimpten Stents notwendig sind. Bevorzugt werden Katheter verwendet, die anstatt eines Ballons einen Bereich eines Innenschafts aufweisen, auf den der selbstexpandierbare Stent zur Applikation gecrimpt werden kann. Zusätzlich weist ein solcher Katheter Mittel auf, die es erlauben den gecrimpten selbstexpandierbaren Stent solange an einer Selbstexpansion zu hindern, bis der Stent an die bestimmte Position gebracht worden ist und dann ein Auslösen der Selbstexpansion des Stents gestatten. Dies kann beispielsweise dadurch erreicht werden, dass der auf den Innenschlauch gecrimpte selbstexpandierbare Stent von einem Außenschlauch umgeben ist, der den Stent an einer Selbstexpansion hindert. Dieser Außenschlauch kann dabei derart ausgestaltet sein, dass ein Zurückziehen des Außenschlauchs im Verhältnis zum Innenschlauch möglich ist, so dass ein Bereich des Innenschlauchs freigesetzt werden kann, auf den der selbstexpandierbare Stent gecrimpt vorliegt. Liegt der Außenschlauch weit genug zurückgezogen vor, so kann der Stent sich aufgrund seiner radialen Kraft vom Innenschlauch lösen, eigenständig expandieren und mit der Gefäßwand in Kontakt treten.

Die erfindungsgemäße Applikationsvorrichtung weist mindestens auf einem Teil der nach außen gewandten Oberfläche des Bereichs der Vorrichtung haarähnliche Fortsätze auf, der zum Crimpen des zu applizierenden Stents vorgesehen ist. Insbesondere können solche Bereiche der Applikationsvorrichtung oder Teile davon mit haarähnlichen Fortsätzen versehen sein, die vor einer Applikation in Kontakt mit einer Oberfläche des zu applizierenden Stents stehen. Bevorzugt weisen mindestens 20% der vom zu applizierenden Stent bedeckbaren Oberfläche haarähnliche Fortsätze auf, besonders bevorzugt weisen 30% bis 100% der vom zu applizierenden Stent bedeckbaren Oberfläche haarähnliche Fortsätze auf. Die haarähnlichen Fortsätze weisen eine Form auf, die auf der Oberfläche der Applikationsvorrichtung fußt und sich darüber erhebt. Insbesondere können die haarähnlichen Fortsätze eine oder mehrere Seitenflächen aufweisen und ggf. eine Oberfläche, die von der Oberfläche der Applikationsform abgewandt ist und die mit einer Oberfläche des zu applizierenden Stents in Kontakt gebracht werden kann. Bevorzugt sind die haarähnlichen Fortsätze im Wesentlichen zylinderförmig.

Die haarähnlichen Fortsätze können grundsätzlich aus jedem Material gefertigt werden, dass sich mit einer vom zu applizierenden Stent bedeckbaren Oberfläche der Applikationsvorrichtung verbinden lässt und das ausreichend verträglich ist für einen Einsatz innerhalb eines menschlichen Körpers. Die haarähnlichen Strukturen bestehen bevorzugt aus Materialien oder Materialzusammensetzungen, die eine Festigkeit und Steifigkeit aufweisen, so dass die haarähnlichen Fortsätze aufrecht von der Oberfläche abstehen können und nicht in sich selbst kollabieren oder abknicken. Die haarähnlichen Fortsätze können insbesondere aus demselben Material gefertigt sein, aus dem die mit einer vom zu applizierenden Stent bedeckbaren Oberfläche der Applikationsvorrichtung gefertigt ist. Geeignete Materialien und Polymere sind dem Fachmann bekannt.

Eine Oberfläche mit haarähnlichen Fortsätzen kann mit unterschiedlichen Verfahren hergestellt werden. Beispielsweise können durch lithographische Verfahren, wie z.B. Elektronenstrahllithographie und Laserlithographie, oder durch Ätzverfahren negative Formen erzeugt werden. In einem anschließenden Gussverfahren wird dann ausgehend von der negativen Form die positive Oberfläche mit haarähnlichen Fortsätzen erzeugt (siehe beispielsweise A.K. Geim et al., Nature Mater. 2, 461-463 (2003) und H. Lee, B.P. Lee and P.B. Messersmith, Nature 448, 338-341 (2007)).

Der durchschnittliche Durchmesser *D* der haarähnlichen Fortsätze, die durchschnittliche Länge *L* der haarähnlichen Fortsätze, sowie der gemittelte Zentrum-zu-Zentrum Abstand *P* der haarähnlichen Fortsätze zueinander sind Parameter, die die Größe der erzielbaren van der Waals-Kräfte beeinflussen.

*D* kann bestimmt werden, in dem für eine ausgewählte Fläche die Durchmesser aller haarähnlichen Fortsätze bestimmt, addiert und die erhaltene Summe durch die Anzahl der vermessenen haarähnlichen Fortsätze geteilt wird.

*L* kann bestimmt werden, in dem für eine ausgewählte Fläche die Länge aller haarähnlichen Fortsätze bestimmt, addiert und die erhaltene Summe durch die Anzahl der vermessenen haarähnlichen Fortsätze geteilt wird.

*P* kann bestimmt werden, in dem für eine ausgewählte Fläche die Abstände aller haarähnlichen Fortsätze zum jeweils nächstgelegenen haarähnlichen Fortsatz bestimmt werden, wobei ausgehend von einem Zentrum eines ersten haarähnlichen Fortsatzes zum Zentrum des nächstgelegenen haarähnlichen Fortsatzes gemessen wird. Die erhaltenen Abstandswerte werden addiert und die erhaltene Summe wird durch die Anzahl der vermessenen haarähnlichen Fortsätze geteilt.

Erfindungsgemäß sind *D, L* und *P* derart ausgewählt, dass ein gegebener Stent auf der mit haarähnlichen Fortsätzen bedeckten Oberfläche der Applikationsvorrichtung fixierbar ist. Auch die Materialeigenschaften des Materials, das zur Herstellung der haarähnlichen Fortsätze eingesetzt wurde, beeinflusst die erzielbaren van der Waals-Kräfte, beispielsweise die Dichte der Materialoberfläche. Die benötigten van der Waals-Kräfte, um einen bestimmten Stent erfindungsgemäß auf der Oberfläche der Applikationsvorichtung zu fixieren, sind nicht für jeden Stent gleich und müssen daher für jede Kombination von Stent und erfindungsgemäßer Applikationsvorrichtung neu bestimmt werden.

Der Fachmann kann ohne Schwierigkeiten durch Routineversuche eine Kombination aus *D, L* und *P* ermitteln, die geeignet ist einen gegebenen Stent in gewünschtem Maße auf der mit haarähnlichen Fortsätzen bedeckten Oberfläche der erfindungsgemäßen Applikationsvorrichtung zu fixieren. Dazu können Testoberflächen bereitgestellt werden, die haarähnliche Fortsätze mit verschiedenen Kombinationen von *D, L* und *P* aufweisen. Diese Testoberflächen können dann mit Oberflächenabschnitten der gewünschten Stents oder den Innenflächen ganzer Stents in Kontakt gebracht werden. Anschließend wird die Kraft bestimmt, die notwendig ist, um die Testoberfläche von der Stentoberfläche zu entfernen.

Insbesondere kann *D* ausgewählt sein aus einem Bereich von 0,05 bis 5 µm, bevorzugt 0,1 bis 4 µm, besonders bevorzugt von 0,2 bis 1 µm. *L* kann insbesondere ausgewählt sein aus einem Bereich von 0,1 bis 20 µm, bevorzugt 0,1 bis 5 µm, besonders bevorzugt von 0,15 bis 2 µm. *P* kann insbesondere ausgewählt sein aus einem Bereich von 0,1 bis 5 µm, bevorzugt von 0,5 bis 3 µm. Bevorzugt sind *D* und *L* derart ausgewählt, dass sich ein Verhältnis von *D* zu *L* ergibt, welches ausgewählt ist aus einem Bereich von 0,5 bis 2, bevorzugt 0,75 bis 1,5. Bevorzugt sind *P* und *D* derart ausgewählt, dass sich ein Verhältnis von *P* zu *D* ergibt, das ausgewählt ist aus einem Bereich von 1 bis 50, bevorzugt von 1,5 bis 10.

Die erfindungsgemäße Applikationsvorrichtung kann haarähnliche Fortsätze aufweisen, wobei die haarähnlichen Fortsätze beschichtet sind mit einem Polymer mit Catechol-Seitengruppen. Diese Polymere erhöhen noch weiter die Haftfähigkeit der haarähnlichen Fortsätze. Geeignete Polymere sowie Polymerzusammensetzungen und Verfahren zur Beschichtung sind in WO 08/091386 offenbart. Solche Polymere können Katecholamine aufweisen, wie Dopamin oder die Aminosäure 3,4-dihydroxy-L-Phenylalanin, auch als DOPA bekannt. Ein besonders bevorzugtes Polymer ist poly(Dopamin Methacrylamid (DMA) - co -Methoxyethyl Acrylat (MEA)). Bevorzugte Polymere weisen Catechol-Seitengruppen auf, wobei die Catechol-Seitengruppen mindestens 5 Gew.% des Gesamtpolymergewichts ausmachen, besonders bevorzugt von 10 Gew.% bis 70 Gew.%. Die Beschichtung der haarähnlichen Fortsätze mit einem Polymer mit Catechol-Seitengruppen kann eine Schichtdicke aufweisen von weniger als 100 nm, bevorzugt ist die Schichtdicke ausgewählt aus einem Bereich von 1 nm bis 50 nm.

Die vorliegende Erfindung bezieht sich auch auf eine Applikationsvorrichtung, die zusätzlich einen applizierbaren Stent, einen applizierbaren, wirkstofffreisetzenden Stent oder einen applizierbaren, biodegradierbaren Stent aufweist. Bevorzugt ist der Stent derart auf der Applikationsvorrichtung angebracht, so dass der Stent applizierbar ist. Insbesondere ist der Stent auf einer dafür vorgesehene und mindestens teilweise in oben beschriebener Weise mit haarähnlichen Fortsätzen ausgestattete Oberfläche der Applikationsvorrichtung angebracht oder gecrimpt. Unter biodegradierbaren Stents werden insbesondere solche Stents verstanden, die biokorrodierbaren Legierungen der Elemente Magnesium, Eisen oder Wolfram für den Grundkörper des Stents aufweisen. Die Legierungen sind so in ihrer Zusammensetzung zu wählen, dass sie biokorrodierbar sind. Als biokorrodierbar im Sinne der Erfindung werden Legierungen bezeichnet, bei denen in physiologischer Umgebung ein Abbau stattfindet, der letztendlich dazu führt, dass der gesamte Stent oder der aus dem Werkstoff gebildete Teil des Stents seine mechanische Integrität verliert. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37 °C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Die vorliegende Erfindung bezieht sich auch auf eine Verwendung einer erfindungsgemäßen Applikationsvorrichtung zur Applikation von Stents, selbstexpandierbaren Stents, wirkstofffreisetzenden Stents und/oder biodegradierbaren Stents, sowie zur Behandlung von Stenosen.

Figuren:
- FIG. 1: zeigt eine beispielhafte Ausführungsform einer erfindungsgemäßen Applikationsvorrichtung als Ballonkatheter.
- FIG. 2: zeigt eine weitere beispielhafte Ausführungsform einer erfindungsgemäßen Applikationsvorrichtung zur Applikation von selbstexpandierbaren Stents.

Die Erfindung wird nachfolgend anhand der Ausführungsbeispiele näher erläutert.

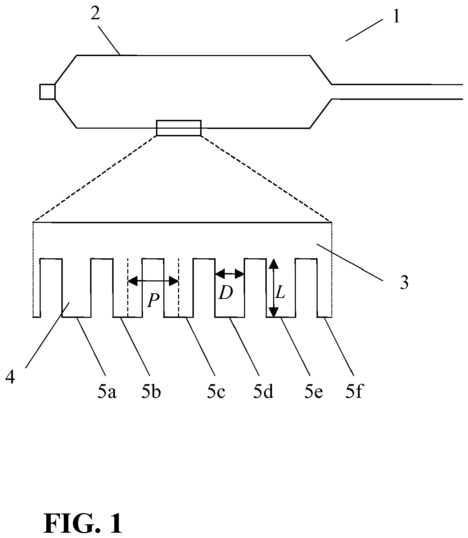

Die in FIG. 1 gezeigte Ausführungsform der erfindungsgemäßen Applikationsvorrichtung ist ein Ballonkatheter 1. Der Ballonkatheter 1 weist einen dilatierbaren Bereich 2 auf. Mindestens auf einem Teil der nach außen gewandten Oberfläche 3 des dilatierbaren Bereichs 2 befindet sich eine Mehrzahl haarähnlicher Fortsätze 4. Diese nach außen gewandten Oberfläche 3 kann nach dem Anbringen oder Crimpen des zu applizierenden Stents auf die Applikationsvorrichtung mit einer Innenoberfläche des Stents in Kontakt stehen und dient der Fixierung des Stents auf der Applikationsvorrichtung. Die haarähnlichen Fortsätze 4 weisen einen bestimmten durchschnittlichen Durchmesser *D*, eine bestimmten durchschnittlichen Länge *L* und einen bestimmten gemittelten Zentrum-zu-Zentrum Abstand *P* auf. Dabei sind *D, L* und *P* so ausgewählt, dass van der Waals-Kräfte zwischen den haarähnlichen Fortsätzen 4 und einer Innenoberfläche eines zu applizierenden Stents zustande kommen, die eine Fixierung des Stents auf der Applikationsvorrichtung erlauben. Die haarähnlichen Fortsätze 4 weisen jeweils eine Oberfläche auf, die mit einer Innenfläche eines zu applizierenden Stents kontaktierbar ist, beispielhaft dargestellt durch 5a bis 5f.

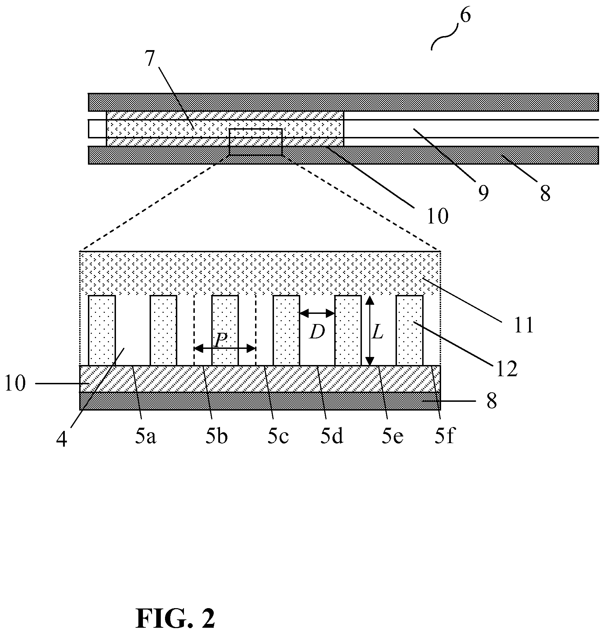

In FIG. 2 ist als weitere erfindungsgemäße Ausführungsform beispielhaft eine Applikationsvorrichtung 6 zur Applikation von selbstexpandierbaren Stents dargestellt. Die Vorrichtung 6 weist einen Innenschlauch 9, einen zurückziehbaren Außenschlauch 8, sowie einen Bereich 7 auf, der zum Anbringen oder Crimpen des zu applizierenden Stents vorgesehen ist. Der Außenschlauch 8 ist derart ausgestaltet, dass er sich im Verhältnis zum Innenschlauch zurückziehen lässt, so dass der Bereich 7 freigelegt werden kann. Im Bereich 7 weist die Vorrichtung 6 einen selbstexpandierbaren Stent 10 auf, der im Bereich 7 zwischen Innenschlauch 9 und Außenschlauch 8 fixiert ist. Mindestens auf einem Teil der nach außen gewandten Oberfläche 11 des Bereichs 7 befindet sich eine Mehrzahl haarähnlicher Fortsätze 4. Diese nach außen gewandte Oberfläche 11 steht zumindest teilweise mit einer Innenoberfläche des Stents 10 in Kontakt und dient der Fixierung des Stents 10 auf der Applikationsvorrichtung 6. Die haarähnlichen Fortsätze 4 weisen einen bestimmten durchschnittlichen Durchmesser *D,* eine bestimmten durchschnittlichen Länge *L* und einen bestimmten gemittelten Zentrum-zu-Zentrum Abstand *P* auf. Dabei sind *D, L* und *P* so ausgewählt, dass van der Waals-Kräfte zwischen den haarähnlichen Fortsätzen 4 und einer Innenoberfläche eines zu applizierenden Stents 10 zustande kommen, die eine Fixierung des Stents 10 auf der Applikationsvorrichtung 6 erlauben. Die haarähnlichen Fortsätze 4 weisen jeweils eine Oberfläche auf, die mit einer Innenfläche eines zu applizierenden Stents 10 kontaktierbar ist, beispielhaft dargestellt durch 5a bis 5f. Zwischen benachbarten haarähnlichen Fortsätzen 4 befindet sich ein Lumen 12. Der Kontakt zwischen Stent 10 und haarähnlichen Fortsätzen 4 des Bereichs 7 führt zu einer Fixierung des Stents auf der Applikationsvorrichtung 6, solange der Stent noch von Teilen des zurückziehbaren Außenschlauchs 8 bedeckt ist. Ist die Applikationsvorrichtung 6 derart platziert, so dass der Stent 10 an die gewünschten Stelle appliziert werden kann, wird der Außenschlauch 8 zurückgezogen, der Stent 10 wird freigelegt, löst sich durch eigenständige radiale Kräfte von Bereich 7 des Innenschlauchs 9 ab und expandiert eigenständig, bis er sich beispielsweise an eine Gefäßwand angelagert hat und dort verbleibt. Nach erfolgter Applikation kann die Vorrichtung 6, nun ohne den applizierten Stent 10, wieder entfernt werden.

### Bezugszeichenliste:

- 1: Ballonkatheter
- 2: dilatierbarer Bereich
- 3: Teil der nach außen gewandten Oberfläche des dilatierbaren Bereichs 2
- 4: haarähnlicher Fortsatz
- 5a bis 5f: Oberfläche des haarähnlichen Fortsatzes, die mit einer Innenfläche eines zu applizierenden Stents kontaktierbar ist
- 6: Applikationsvorrichtung zur Applikation von selbstexpandierbaren Stents
- 7: Bereich der zum Anbringen oder Crimpen des zu applizierenden Stents vorgesehen ist
- 8: zurückziehbarer Außenschlauch
- 9: Innenschlauch
- 10: selbstexpandierbarer Stent
- 11: nach außen gewandte Oberfläche des Bereichs 7
- 12: Lumen zwischen benachbarten haarähnlichen Fortsätzen

## Patentansprüche

1. Applikationsvorrichtung für Stents (6), wobei die Applikationsvorrichtung mindestens auf einem Teil der nach außen gewandten Oberfläche (3) eines Bereichs der Vorrichtung haarähnliche Fortsätze (4) aufweist, **dadurch gekennzeichnet dass** der Bereich zum Anbringen oder Crimpen des zu applizierenden Stents vorgesehen ist, wobei der durchschnittliche Durchmesser *D,* die durchschnittliche Länge *L* und der gemittelte Zentrum-zu-Zentrum-Abstand *P* der haarähnlichen Fortsätze (4) zueinander derart ausgewählt sind, dass ein Stent auf der mit haarähnlichen Fortsätzen (4) bedeckten Oberfläche im Wesentlichen mittels van der Waals-Kräften fixierbar ist.

2. Applikationsvorrichtung (6) nach Anspruch 1, wobei die Applikationsvorrichtung einen Katheter umfasst.

3. Applikationsvorrichtung (6) nach Anspruch 1 oder 2, wobei die Applikationsvorrichtung (6) eine dilatierbare Ballonoberfläche (2) aufweist, auf die ein Stent gecrimpt werden kann.

4. Applikationsvorrichtung (6) nach Anspruch 1 oder 2, wobei die Applikationsvorrichtung (6) eine Oberfläche zur Applikation selbstexpandierbarer Stents aufweist.

5. Applikationsvorrichtung (6) nach einem der vorhergehenden Ansprüche, wobei die haarähnlichen Fortsätze (4) im Wesentlichen zylinderförmig sind.

6. Applikationsvorrichtung (6) nach einem der vorhergehenden Ansprüche, wobei *D* ausgewählt ist aus einem Bereich von 0,05 bis 5 µm.

7. Applikationsvorrichtung (6) nach einem der vorhergehenden Ansprüche, wobei *L* ausgewählt ist aus einem Bereich von 0,1 bis 20 µm.

8. Applikationsvorrichtung (6) nach einem der vorhergehenden Ansprüche, wobei *P* ausgewählt ist aus einem Bereich von 0,1 bis 5 µm, bevorzugt von 0,5 bis 3 µm.

9. Applikationsvorrichtung (6) nach einem der vorhergehenden Ansprüche, wobei das Verhältnis *D* zu *L* ausgewählt ist aus einem Bereich von 0,5 bis 2, bevorzugt 0,75 bis 1,5.

10. Applikationsvorrichtung (6) nach einem der vorhergehenden Ansprüche, wobei das Verhältnis *P* zu *D* ausgewählt ist aus einem Bereich von 1 bis 50, bevorzugt von 1,5 bis 10.

11. Applikationsvorrichtung (6) nach einem der vorhergehenden Ansprüche, wobei die haarähnlichen Fortsätze (4) beschichtet sind mit einem Polymer mit Catechol-Seitengruppen.

12. Applikationsvorrichtung (6) nach Anspruch 11, wobei die Beschichtung eine Schichtdicke aufweist von weniger als 100 nm, bevorzugt ist die Schichtdicke ausgewählt aus einem Bereich von 5 nm bis 50 nm.

13. Applikationsvorrichtung (6) nach einem der vorhergehenden Ansprüche, wobei die Applikationsvorrichtung (6) zusätzlich einen applizierbaren Stent, einen applizierbaren, wirkstofffreisetzenden Stent oder einen applizierbaren, biodegradierbaren Stent aufweist.

## Claims

1. Implantation device for stents (6), wherein the device has hair-like extensions (4), at least on one part of the outwardly-facing surface (3) of that section of the device, **characterized in that** the section is provided for the attachment or crimping of the stent to be implanted, wherein the mean diameter *D,* the mean length *L,* and averaged center-to-center distance P of the hair-like extensions (4) relative to each other are selected such that a stent is essentially affixable by van der Waals forces on the surface covered with hair-like extensions (4).

2. Implantation device (6) according to claim 1, wherein the implantation device comprises a catheter.

3. Implantation device (6) according to claim 1, wherein the implantation device (6) has a dilatable balloon surface (2) onto which a stent can be crimped.

4. Implantation device (6) according to claim 1, wherein the implantation device (6) has a surface for implantation of self-expanding stents.

5. Implantation device (6) according to claim 1, wherein the hair-like extensions (4) are essentially cylindrical.

6. Implantation device (6) according to claim 1, wherein *D* is selected from a range between 0.05 and 5 µm.

7. Implantation device (6) according to claim 1, wherein *L* is selected from a range between 0.1 and 20 µm.

8. Implantation device (6) according to claim 1, wherein *P* is selected from a range between 0.1 and 5 µm, preferably, between 0.5 and 3 µm.

9. Implantation device (6) according to claim 1, wherein the ratio of *D* to *L* is selected from a range between 0.5 and 2, preferably, 0.75 and 1.5.

10. Implantation device (6) according to claim 1, wherein the ratio of *P* to *D* is selected from a range between 1 and 50, preferably, 1.5 and 10.

11. Implantation device (6) according to claim 1, wherein the hair-like extensions (4) are coated with a polymer having catechol side groups.

12. Implantation device (6) according to claim 11, wherein the coating has a coating thickness of less than 100 nm; preferably, the coating thickness is selected from a range between 5 nm and 50 nm.

13. Implantation device (6) according to claim 1, wherein the implantation device (6) additionally has an implantable stent, an implantable pharmaceutical-agent-releasing stent, or an implantable biodegradable stent.

## Revendications

1. Dispositif d'implantation pour stents (6), le dispositif d'implantation présentant, au moins sur une partie de la surface (3) orientée vers l'extérieur d'une région du dispositif, des extensions (4) semblables à des cheveux, **caractérisé en ce que** la région est prévue pour l'implantation ou l'agrippement du stent à implanter, où le diamètre moyen *D,* la longueur moyenne *L* et la distance de centre à centre moyennée *P* des extensions (4) semblables à des cheveux sont choisis les uns par rapport aux autres de telle manière qu'un stent peut être fixé sur la surface recouverte d'extensions (4) semblables à des cheveux essentiellement au moyen de forces de Van der Waals.

2. Dispositif d'implantation (6) selon la revendication 1, où le dispositif d'implantation comprend un cathéter.

3. Dispositif d'implantation (6) selon la revendication 1 ou la revendication 2, où le dispositif d'implantation (6) présente une surface de ballon (2) pouvant se dilater sur laquelle un stent peut être agrippé.

4. Dispositif d'implantation (6) selon la revendication 1 ou la revendication 2, où le dispositif d'implantation (6) présente une surface pour l'implantation d'un stent auto-expansif.

5. Dispositif d'implantation (6) selon l'une des revendications précédentes, dans lequel les extensions (4) semblables à des cheveux sont essentiellement de forme cylindrique.

6. Dispositif d'implantation (6) selon l'une des revendications précédentes, dans lequel *D* est choisi dans une plage de 0,05 à 5 µm.

7. Dispositif d'implantation (6) selon l'une des revendications précédentes, dans lequel L est choisie dans une plage de 0,1 à 20 µm.

8. Dispositif d'implantation (6) selon l'une des revendications précédentes, dans lequel *P* est choisie dans une plage de 0,1 à 5 µm, de préférence de 0,5 à 3 µm.

9. Dispositif d'implantation (6) selon l'une des revendications précédentes, dans lequel le rapport *D* sur *L* est choisi dans une plage de 0,5 à 2, de préférence, de 0,75 à 1,5.

10. Dispositif d'implantation (6) selon l'une des revendications précédentes, dans lequel le rapport *P* sur *D* est choisi dans une plage de 1 à 50, de préférence, de 1,5 à 10.

11. Dispositif d'implantation (6) selon l'une des revendications précédentes, dans lequel les extensions (4) semblables à des cheveux sont revêtues avec un polymère comportant des groupes latéraux catéchols.

12. Dispositif d'implantation (6) selon la revendication 11, dans lequel le revêtement présente une épaisseur de couche inférieure à 100 nm, l'épaisseur de couche est de préférence choisie dans une plage de 5 nm à 50 nm.

13. Dispositif d'implantation (6) selon l'une des revendications précédentes, où le dispositif d'implantation (6) présente en outre un stent implantable, un stent implantable libérant une matière active ou un stent implantable biodégradable.
